# Europäisches Patentamt

# European Patent Office

# Office européen des brevets

(11) Veröffentlichungsnummer: **0 117 519**
**A1**

(12) ## EUROPÄISCHE PATENTANMELDUNG

(21) Anmeldenummer: **84101828.6**

(22) Anmeldetag: **22.02.84**

(51) Int. Cl.³: **A 61 B 17/22,** A 61 B 17/32

(30) Priorität: **23.02.83 DE 3306213**
**10.06.83 DE 3320984**

(43) Veröffentlichungstag der Anmeldung: **05.09.84**
**Patentblatt 84/36**

(84) Benannte Vertragsstaaten: **AT BE CH FR GB IT LI NL SE**

(71) Anmelder: **Theermann, Johannes, Dipl.-Ing.,**
**Ruhrallee 28, D-4300 Essen 1 (DE)**

(72) Erfinder: **Theermann, Johannes, Dipl.-Ing., Ruhrallee 28,**
**D-4300 Essen 1 (DE)**

(74) Vertreter: **Spalthoff, Adolf, Dipl.-Ing.,**
**Pelmanstrasse 31 P.O. Box 34 02 20,**
**D-4300 Essen 1 (DE)**

(54) **Katheter.**

(57) Um einen Katheter zu schaffen, welcher nicht nur in einfacher Weise herzustellen und zu handhaben ist, sondern darüber hinaus eine einwandfreie und vollständige Ausräumung von Einschlüssen in einer für den Patienten gefahrlosen Weise ermöglicht, wird erfindungsgemäß vorgeschlagen, daß sich am vorderen Ende des Rohrkörpers (1) ein Spreizkopf (2) befindet, welcher ein oder mehrere Schälmesser (6) aus hochelastischem Material aufweist, welches bzw. welche mittels eines in dem Rohrkörper (1) verlaufenden Spanndrahtes (7) od. dgl. auswölbbar ist bzw. sind und am hinteren Ende (10) des Rohrkörpers (1) ein Griff (5) vorhanden ist. Um sicherzustellen, daß von einer Verkalkung nicht betroffene Gefäßwandungen nicht mit den rotierenden Schälmessern in Berührung gelangen können, wird weiterhin vorgeschlagen, daß der Spreizkopf (2) mit einem etwa bandförmigen Schutzschild aus hochelastischem Material ausgerüstet ist, welcher sich über einen Teil des Umfanges des Spreizkopfes erstreckt. Um einen Blutverlust weitgehend zu vermeiden wird weiterhin vorgeschlagen, daß auf dem Rohrkörper (1) in seinem Bereich vor den Schälmessern (6) und/oder auf dem Spanndraht (7) in seinem Bereich zwischen den beiden Teilen der geteilt ausgebildeten Spitze (3) des Spreizkopfes (2) ein Schlauchstück aus elastischem Material angebracht ist bzw. sind, in dessen bzw. deren Überdeckungsbereiche eine oder mehrere Austrittsöffnungen von Kanälen münden, die die Wandungen des Rohrkörpers (1) und/oder des Spanndrahtes (7) in deren Längsrichtung durchsetzen.

"Katheter"

Die Erfindung betrifft ein Katheter zum Ausräumen von Einschlüssen, wie Thromben, Tumoren, sklerotischen Herden und Verstopfungen in Venen, Arterien, Luft- und Speiseröhren sowie anderen Hohlorganen des Körpers, welches einen biegsamen Rohrkörper aus elastisch weichem Material aufweist.

Es ist bekannt, daß schwere Durchblutungsstörungen in den Gliedmaßen, besonders in den Beinen, dazu führen, daß Jahr für Jahr einer Vielzahl von Patienten ein Bein amputiert werden muß oder aber starke Behinderungen des Bewegungsmechanismus eintreten. Diese Leiden sind vielfach auf Kreislauf- und Durchblutungsstörungen zurückzuführen, welche durch Einlagerungen, fetter, verkalkender Ablagerungen in der Gefäßwand (Sklerosen) hervorgerufen werden, die zunächst zu einer Verengung und später zur völligen Verstopfung des Gefäßes führen. (Thromben, Stenosen)

Es ist versucht worden, durch gerinnungslösende Medikamente Abhilfe zu schaffen, jedoch bestehen Zweifel an dem Erfolg dieser Behandlungsmethode. So bleibt für die Behandlung besonders schwerer Fälle nur die Obliteration (Verödung) oder die Bypass-Operation durch ein Gefäßtransplantat. Diese schweren Eingriffe, die mit einem nicht unerheblichen Risiko verbunden sind, werden in besonders schweren Fällen sicherlich immer durchgeführt werden müssen. In letzter Zeit gehen die Bestrebungen jedoch dahin, dem Leiden ohne komplizierte Eingriffe beizukommen, und zwar mit Hilfe von Kathetern, um ein Ausräumen der Einschlüsse zu ermöglichen.

- 2 -

Es ist bekannt, zur Ausräumung des Gefäßvolumens die verschlossene Ader an einer gut zugänglichen Stelle freizulegen und den Thrombus mit einem Fogartykatheter oder einem
Ringkatheter herauszuziehen. Der Fogartykatheter, der einen
Schlauchkatheter darstellt, wird durch den Thrombuswulst
hindurchgestoßen, woraufhin hinter dem Thrombuswulst die
Spitze des Schlauchkatheters zu einem Ballon aufgepumpt
und damit der Thrombus aus der Ader herausgezogen wird.
Der Ringkatheter hat am Ende eines Rohres eine Schlinge aus
Draht, die um den Wulst gelegt wird, woraufhin dieser herausgezogen wird. Beide bekannten Ausführungsformen erweisen
sich jedoch als nachteilig, weil es nicht immer gelingt,
den Thrombus als Ganzes zu extrahieren und sklerotische Einlagerungen nicht erfaßt werden können.

Es ist weiterhin bekannt, zum Aufweiten von Verengungen ein
Ballonkatheter zu verwenden. Das vordere Ende dieses Katheters
wird bis in die Verengung vorgeschoben, woraufhin das Ende
ballonförmig oder wulstförmig aufgepumpt wird, wobei Drücke
bis zu 5 bar auftreten können. Der aderverengende Belag wird
auf diese Weise mechanisch zusammengedrückt bzw. zurückgepreßt, so daß ein neuer Kanal entsteht. Als nachteilig hat
sich gezeigt, daß diese Maßnahme nicht von langer Dauer ist,
weil die Preßlage zum Teil wieder zurückfedert und sich neue
Ablagerungen bilden. Nicht anwendbar ist diese Methode bei
längeren Gefäßabschnitten. Auch erweist es sich als nachteilig, daß bei dieser Dilatation der Blutstrom für einige
Zeit völlig blockiert werden muß, mit allen daraus resultierenden Risiken.

Es ist auch schon vorgeschlagen worden, ein Blutgerinnsel mit einem als Drahtausgebildeten Katheter zu durchlöchern und zu durchstoßen. Die Gefahr, daß auf diese Weise gelöste Partikel mit dem Blutstrom weitergeleitet werden und Embolien verursachen, ist sehr groß, so daß diese Methode als wenig brauchbar angesehen werden muß.

Fernerhin ist ein Katheter bekannt, der speziell in der Gynäkologie zur Durchführung von Gebärmutterabstrichen einsetzbar ist. Dieser besteht aus einem kurzen Plastikrohr, in dem ein zweites Rohr verschiebbar angeordnet ist, welches vorne gespalten ist, so daß nach Vorschieben des inneren Rohres aus dem äußeren Rohr der gespaltene Teil sich durch eigene Elastizität aufwölbt. Mit Hilfe der Aufwölbungen kann durch Drehung von Hand an dem inneren Rohr ein leichter Schabeeffekt erreicht werden, um Gewebeproben od.dgl. zur Durchführung mikroskopischer Untersuchungen zu entnehmen.

Sämtlichen vorbekannten Kathetern und den durch deren Ausführungsart herrührenden Arbeitsweisen haftet der Nachteil an, daß abhängig von der jeweiligen Plastizität der Thromben diese den Einschluß nicht oder nur teilweise erfassen, bei Verhärtungen aber völlig versagen und bei weicheren Thromben diese vor sich her in die Blutbahn geschoben werden, was die Gefahr von Embolien mit sich bringt, so daß der Einsatz dieser bekannten Katheter sehr fragwürdig ist.

Von diesem Stand der Technik ausgehend liegt der Erfindung die Aufgabe zugrunde, unter Vermeidung vorerwähnter Nachteile einen Katheter der eingangs genannten Art zu schaffen, welcher nicht

- 4 -

nur in einfacher Weise herzustellen und zu handhaben ist,
sondern darüber hinaus eine einwandfreie und vollständige Ausräumung von Einschlüssen in einer für den Patienten gefahrlosen Weise ermöglicht.

Gemäß der Erfindung wird dies dadurch erreicht, daß sich am
vorderen Ende des Rohrkörpers ein Spreizkopf befindet, welcher ein oder mehrere Schälmesser aus hochelastischem Material aufweist, welches bzw. welche mittels eines in dem
Rohrkörper verlaufenden Spanndrahtes od.dgl. auswölbbar ist bzw.
sind, und am hinteren Ende des Rohrkörpers ein Griff vorhanden ist. Durch diese Ausgestaltung ist es möglich, etwaige
Einschlüsse, gleich ob diese weich, knorpelig, fest oder versteint sind, zu zerstören, wobei der Grad der Auswölbung der
Messer in einfacher Weise einstellbar ist, wobei dann eine Anpassung an den inneren Durchmesser der Gefäße usw. möglich ist.

Der Spanndraht od.dgl. erstreckt sich von der Spitze des Spreizkopfes bis zum Griff, so daß vom Griff aus durch mehr oder weniger starkes Anziehen des Spanndrahtes der Grad der Wölbung der
Schälmesser einstellbar ist.

Das bzw. die Schälmesser stützt bzw. stützen sich mit ihren
Enden einerseits an der Spitze und andererseits an einem Endstück des Spreizkopfes ab. Da die Krümmung der Schälmesser kraftschlüssig erfolgt, verbleiben sie in dieser Lage formstabil, auch
bei sehr großem Gegendruck durch die Innenwand der Gefäße, so daß
die schabende Wirkung voll erhalten bleibt. Sobald die auf den
Spanndraht einwirkende Zugkraft verringert wird, kehren die

- 5 -

Schälmesser infolge ihrer starken Elastizität in ihre Ausgangslage zurück, in welcher sie bündig im Spreizkopf liegen.

Gemäß einem weiteren Merkmal der Erfindung ist im Rohrkörper ein schraubenlinienförmig gewickeltes Band vorgesehen, welches sich von der Spitze des Spreizkopfes bis zum Griff erstreckt. Dieses schraubenlinienförmige Band dient der Abförderung der durch die Schälmesser gelösten Partikel, und zwar rückwärts durch den Rohrkörper, bis in den Bereich des Griffes, so daß die gelösten Partikel nicht in die Blutbahn gelangen können und damit die Gefahr von Embolien weitgehend ausgeschlossen ist. Das schraubenlinienförmige Band umgibt vorteilhaft den Spanndraht, so daß sich eine kompakte Bauweise ergibt.

Gemäß einem weiteren Merkmal der Erfindung sind die vorderen Enden des Spanndrahtes od.dgl. und des schraubenlinienförmigen Bandes an der Spitze des Spreizkopfes drehfest und deren hintere Enden am Griff drehbar angeordnet. Infolge ihrer koaxialen Anordnung und Befestigung ihrer Enden aneinander führen diese zusammen mit dem Spreizkopf und dessen Schälmessern eine Drehbewegung aus, so daß beim Abschälen von Einschlüssen diese gleichzeitig zurückgefördert werden.

Im Griff befindet sich ein Elektromotor, ggf. mit Batterie oder mit einer Anschlußmöglichkeit an das Netz, so daß die Bedienung mit einer Hand vom Griff aus möglich ist.

Nach einem weiteren Vorschlag der Erfindung mündet das hintere Ende des Rohrkörpers in ein mit Außengewinde versehenes Rohrstück ein, welches von einem Rohrstutzen übergriffen ist, welcher mit einer Gewindemuffe versehen ist und über ein Zwi-

- 6 -

schenstück an den Griff angeschlossen ist. Durch Verdrehen der Gewindemuffe wird das innere Rohrstück mehr
oder weniger weit in den Rohrstutzen hineingezogen oder
aber aus diesem herausgeschoben, was eine Spannung bzw.
Entspannung des Spanndrahtes zur Folge hat, da der Griff
je nach dem Grad der Verdrehung der Gewindemuffe entweder dem Ende des Rohrkörpers angenähert oder aber von
diesem entfernt wird. Rohrstück und Rohrstutzen sind über
eine Nut miteinander gekoppelt, so daß diese relativ zueinander keine Dreh- sondern nur eine Längsbewegung ausführen.

Das Zwischenstück weist in seiner Wandung eine oder mehrere
Durchtrittsöffnungen auf, so daß die zurückgeförderten Partikel hier austreten können. Es ist jedoch auch möglich, das
Zwischenstück mit einem Anschlußstutzen auszurüsten, so daß
eine Vakuumpumpe angeschlossen werden kann, welche die zurückgeförderten Partikel laufend absaugt.

Um sicherzustellen, daß von einer Verkalkung od.dgl. nicht
betroffene Gefäßwandungen nicht mit den rotierenden Schälmessern in Berührung gelangen können, wird erfindungsgemäß
vorgeschlagen, daß der Spreizkopf mit einem etwa bandförmigen
Schutzschild aus hochelastischem Material ausgerüstet ist,
welcher sich über einen Teil des Umfanges des Spreizkopfes erstreckt, beispielsweise ein Viertel oder ein Drittel. Der durch
den Schutzschild abgedeckte Bereich des Spreizkopfes wird bei
einseitigen Einschlüssen zu der von einer Verkalkung nicht betroffenen Gefäßwand hingedreht, so daß diese durch den Schutzschild abgedeckt ist und nicht mit den rotierenden Schälmessern in
Berührung gelangen kann. Diese beseitigen lediglich die einseitigen Einschlüsse, welche nach hinten hin wegbefördert werden. Bei wechselnden Wandveränderungen in den verschiedenen Gefäßabschnitten kann die Position der Wandabschirmung des Katheters durch Drehen des Rohres jeweils auf den gesunden zu schützenden Teil der Gefäßwand ausgerichtet werden.

Vorteilhaft ist das eine Ende des Schutzschildes fest mit einem Rohrstück verbunden, welches das Endstück des Spreizkopfes übergreift und an dem vorderen Ende des Rohrkörpers befestigt ist. Das Rohrstück ist somit am vorderen Ende des Rohrkörpers des Katheters festgelegt, so daß dieses die Drehbewegung nicht mit auszuführen vermag und damit auch nicht der hieran befestigte Schutzschild.

Gemäß einem weiteren Merkmal der Erfindung weist das andere Ende des Schutzschildes eine Abbiegung senkrecht zur Katheterachse auf, welche Abbiegung sich in einem Spalt zwischen den Teilen der geteilt ausgebildeten Spitze des Spreizkopfes erstreckt und von dem Spanndraht durchsetzt ist. Die Breite des Spaltes ist derart festgelegt, daß die Abbiegung des Schutzschildes hierin mit Spiel untergebracht ist, so daß die geteilte Spitze gegenüber dem Schutzschild ihre für die Funktion der Schälmesser wichtige Drehbewegung ausführen kann.

Der eine Teil der Spitze ist von einer Gewindemuffe gebildet und der andere Teil von einem mit Innengewinde versehenen Zylinder. Beide Teile sind auf das Ende des Spanndrahtes unter Belassung eines Spaltes aufgeschraubt. Zu diesem Zwecke ist das Ende des Spanndrahtes mit Außengewinde ausgerüstet.

- 8 -

Für den Fall, daß Stenosen den ganzen Querschnitt eines
Gefäßes ausfüllen besteht die Gefahr, daß die Schälmesser des Spreizkopfes diese nicht erfassen können. Um auch
in einem solchen Fall ein Ausräumen der Einschlüsse zu
ermöglichen, ist nach einem weiteren Merkmal der Erfindung die Spitze des Spreizkopfes auf ihrer Umfläche mit
fräserartigen Rillen od.dgl. versehen. Durch diese Ausgestaltung wird es möglich, daß der Spreizkopf sich zuerst ein
mittiges Loch in der Größe seines Durchmessers schafft, woraufhin die Schälmesser zum Einsatz gelangen können. Die fräserartigen Rillen od.dgl. verlaufen schräg zur Katheterachse,
so daß die gelösten Partikel nach rückwärts transportiert
werden.

Um einen Blutverlust bei Anwendung des erfindungsgemäßen Katheters weitgehend zu vermeiden, wird weiterhin vorgeschlagen,
daß auf dem Rohrkörper in seinem Bereich vor den Schälmessern
und/oder auf dem Spanndraht in seinem Bereich zwischen den beiden Teilen der geteilt ausgebildeten Spitze des Spreizkopfes
ein Schlauchstück aus elastischem Material angebracht ist bzw.
sind, in dessen bzw. deren Überdeckungsbereiche eine oder
mehrere Austrittsöffnungen von Kanälen münden, die die Wandung des Rohrkörpers und/oder des Spanndrahtes in deren Längsrichtungen durchsetzen. Vorteilhaft ist der bzw. sind die Kanäle an eine Druckmittelquelle anschließbar.

Durch die erfindungsgemäße Ausgestaltung ist es möglich, durch
Druckmittelbeaufschlagung die elastischen Schläuche ballonartig aufzuwölben und mit den Innenwandungen der Venen, Arterien

- 9 -

usw. zu verspannen, so daß die Ausräumstelle abgedichtet
ist und ein Blutverlust an dem Rohrkörper und/oder dem
Spanndraht vorbei weitgehend unterbunden ist.

Je nach Lage und Form der Thromben, sklerotischen Stenosen
usw. können der vordere oder nur der hintere elastische
Schlauch zur Abdichtung verwendet werden oder aber auch
beide gleichzeitig. Durch Einsatz des bzw. der ballonartig ausgewölbten Schlauchstücke wird die Ausräumstelle vor
und/oder hinter den Schälmessern vorübergehend gegen den
Blutstrom abgedichtet, so daß sich ein absatzweises Arbeiten ergibt.

Da die Venenwandung wesentlich dünner und weniger widerstandsfähig ist als die der Arterien und außerdem weniger Haftung
in dem sie umgebenden Faszienschlauch besitzt, wird durch
Aufpumpen der beiden ballonartigen Schlauchstücke die Vene
nach außen an den Faszienschlauch bzw. das Muskelgewebe angedrückt und auf diese Weise bewirkt, daß sie dort festsitzt
und sich nicht mitdrehen kann. Außerdem wird erreicht, daß
der Blutverlust sich nur auf die zwischen den ballonartig
aufgeblasenen Schlauchstücken befindliche Venenpartie erstrecken kann.

Ausführungsbeispiele der Erfindung sind an Hand der Zeichnung
näher erläutert, und zwar zeigt:

Figur 1    eine Ansicht des erfindungsgemäßen Katheters
           mit geschlossenem Spreizkopf,

- 10 -

Figur 2  eine Ansicht des geöffneten Spreizkopfes,

Figur 3  eine geschnittene Seitenansicht des Spreizkopfes gemäß der Linie III - III der Figur 2,

Figur 4  eine Ansicht des vorderen Teils des Griffes,

Figur 5  eine Ansicht des Spreizkopfes in einer weiteren
Ausführung mit gewölbter Position der Schälmesser,

Figur 6  eine geschnittene Ansicht des Spreizkopfes nach

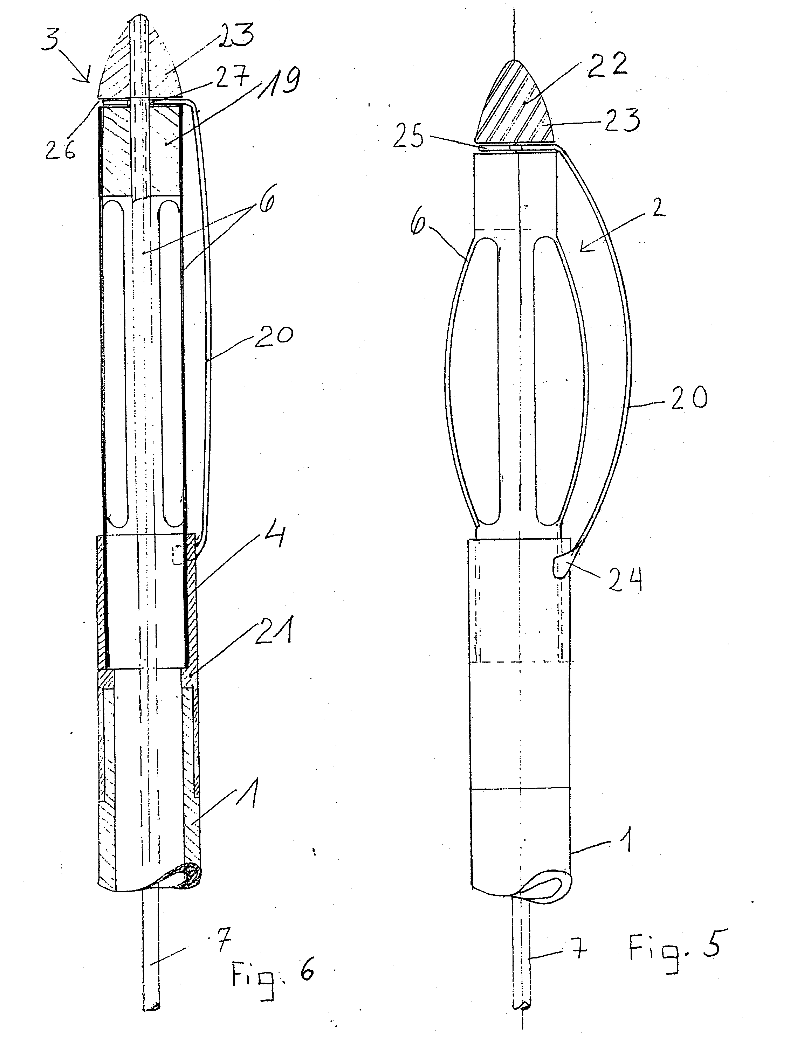

Figur 5 mit in ihrer Ruhelage befindlichen
Schälmessern,

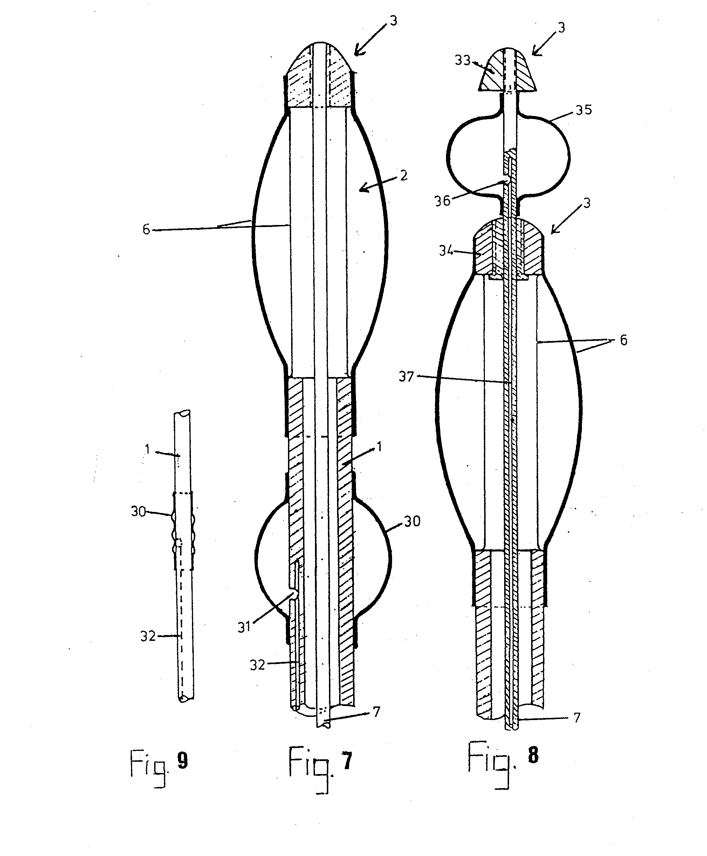

Figur 7  eine geschnittene Ansicht einer weiteren Ausführung des Spreizkopfes mit einem Schlauchstück unterhalb der Schälmesser,

Figur 8  eine geschnittene Ansicht des Spreizkopfes mit
einer Anordnung eines Schlauchstückes zwischen den
beiden Teilen der Spitze und

Figur 9  in schematischer Darstellung einen Abschnitt des
Rohrkörpers mit aufgebrachtem Schlauchstück.

Mit 1 ist der Rohrkörper des Katheters bezeichnet, welcher aus
einem elastisch weichen, biegsamen Kunststoff hergestellt ist.
Am vorderen Ende des Rohrkörpers 1 befindet sich ein Spreizkopf
2, dessen Spitze mit 3 und dessen Endstück mit 4 bezeichnet ist.
Am hinteren Ende des Rohrkörpers 1 ist der Griff 5 vorgesehen.

Der Spreizkopf 2 weist mehrere Schälmesser 6, im dargestellten
Ausführungsbeispiel vier, auf, deren Enden sich zwischen der
Spitze 3 einerseits und dem Endstück 4 abstützen.

Im Rohrstück 1 befindet sich ein Spanndraht 7 sowie ein schrau-

- 11 -

benlinienförmig gewickeltes Band 8, deren Anordnung derart
ist, daß das schraubenlinienförmig gewickelte Band 8 den
Spanndraht 7 umgreift, d.h. beide sind koaxial angeordnet.
Die vorderen Enden des Spanndrahtes 7 sowie des Bandes 8
sind drehfest mit der Spitze 3 verbunden, während deren
hintere Enden mit der Welle 9 des im Griff angeordneten
Elektromotors verbunden sind. Der Spreizkopf 2 ist über
das Endstück 4 drehbar gegenüber dem Rohrkörper 1.

Das hintere Ende 10 des Rohrkörpers 1 mündet in ein mit
Außengewinde versehenes Rohrstück 11 ein, das von einem
Rohrstutzen 12 übergriffen ist. Auf dem Rohrstutzen 12 befindet
sich eine Schraubmuffe 13, die auf das Außengewinde des Rohrstückes 11 aufgeschraubt ist. Durch Verdrehen der Gewindemuffe
13 wird das Gehäuse 5 entweder an das Ende 10 des Rohrkörpers
11 heranbewegt oder aber von diesem wegbewegt, so daß je
nach Drehsinn entweder der im Rohrkörper 1 verlaufende Spanndraht 7 gespannt oder aber entspannt wird.

Der Rohrstutzen 12 geht über das Zwischenstück 18 in den eigentlichen Griff 5 über. Das Zwischenstück weist in seiner Wandung
Durchtrittsöffnungen 14 auf, welche zur Ableitung der von den
Schälmessern 6 gelösten Partikel dienen.

Die Wirkungsweise des erfindungsgemäßen Katheters ist wie folgt:
Nach Einführen des Spreizkopfes 2 des Katheters in ein Gefäß
des menschlichen Körpers wird dieser bis zum Einschluß vorgeschoben. Durch geringfügiges Verdrehen der Gewindemuffe 13
wird der Spanndraht 7 angezogen, so daß die Spitze 3 des Spreizkopfes 2 gegen das Endstück 4 hin bewegt wird unter
gleichzeitiger Auswölbung der Schälmesser 6. Durch Einschalten des Schalters 15 wird der Elektromotor in Drehung
versetzt und über dessen Kupplung 9 der Spanndraht 7 zusammen
mit dem schraubenlinienförmigen Band 8. Die von den Schälmessern 6 gelösten Partikel werden über das Band 8 rückwärts durch den Rohrkörper bis in das Zwischenstück 18
gefördert, aus dessen Durchtrittsöffnungen 14 diese austreten können. Nachdem auf diese Weise durch die Einlagerung ein durchgängiger Kanal hergestellt worden ist,
wird der Spanndraht 7 weiter angezogen durch Drehen der
Gewindemuffe 13, so daß die Schälmesser 6 weiter nach außen
gewölbt werden und die nächste Lage abgeschält werden kann.
Hierbei wird der Spreizkopf immer hin und her bewegt, so daß
die gesamte Einlagerung auch über einen größeren Gefäßabschnitt
Lage für Lage abgeschält und ausgeräumt wird.

Die kraftschlüssigerzeugte Krümmung der Schälmesser 6 ist
formstabil und bleibt auch gegen größeren Druck der Gefäßinnenwände schälwirksam erhalten.

Im Griff 5 können Batterien für den Elektromotor vorgesehen
sein. Es ist jedoch auch möglich, diesen mittels der Schnur
16 vom Netz her zu betreiben, ggf. unter Zwischenordnung eines
Gleichrichters.

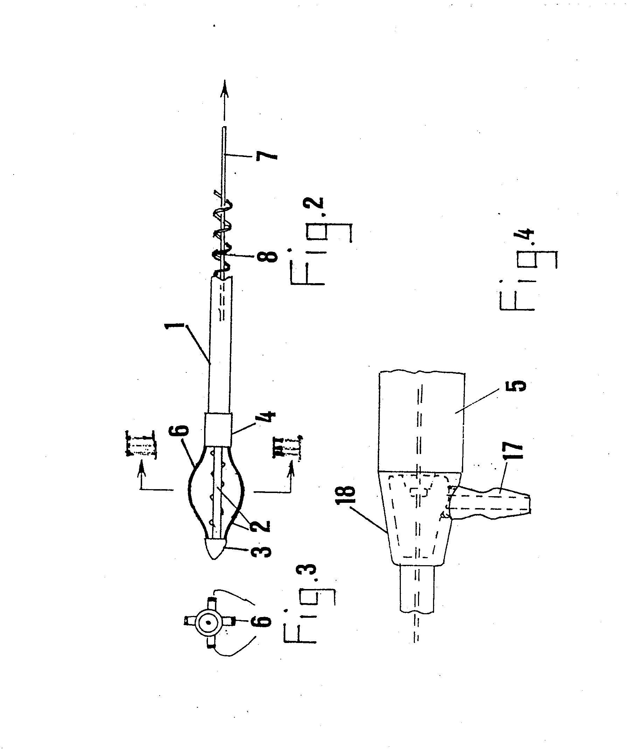

Wie aus Figur 2 und 3 hervorgeht, werden durch Verkürzung des
Abstandes zwischen Spitze 3 und Endstück 4 die Schälmesser 6
mehr oder weniger stark ausgewölbt, so daß eine Einstellbarkeit auf die gewünschten Innendurchmesser der Gefäße in ein-

- 13 -

facher Weise durchführbar ist.

In Figur 4 ist gezeigt, daß das Zwischenstück 18 auch mit
einem Anschlußstutzen 17 ausgerüstet sein kann, um eine Vakuumpumpe zur Absaugung der gelösten Partikel anzuschliessen.

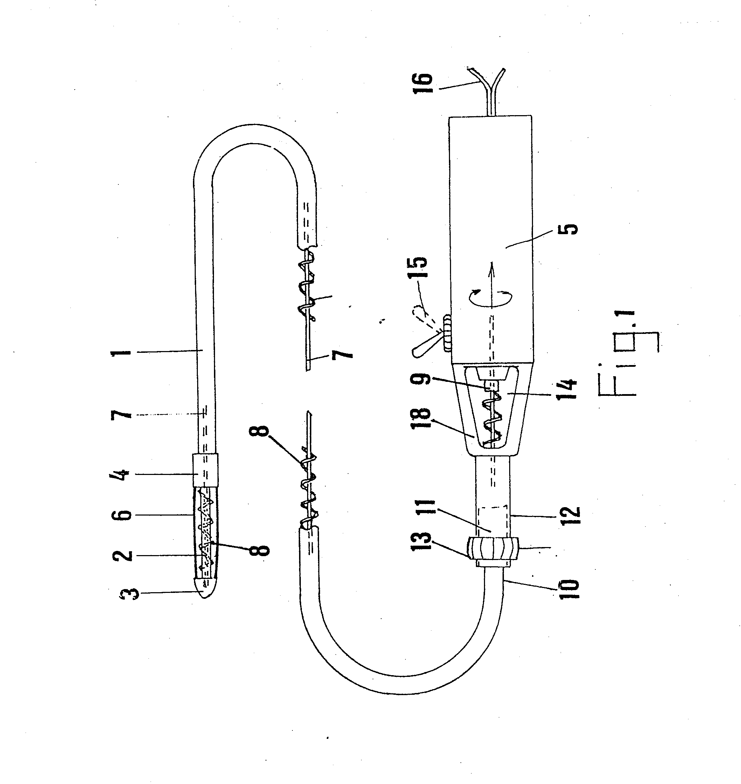

Bei der Ausführung nach Figur 5 und 6 ist der Spreizkopf
2 mit einem etwa bandförmigen Schutzschild 20 ausgerüstet,
welcher sich über einen Teil des Umfanges des Spreizkopfes 2 erstreckt, beispielsweise über ein Viertel oder
ein Drittel des Umfanges. Das eine Ende 24 des Schutzschildes 20 ist fest mit einem Rohrstück 21 verbunden, und
zwar auf dessen Außenseite, wie insbesondere Figur 1 erkennen läßt. Das Rohrstück 21 übergreift das Endstück 4,
welches in dem Rohrstück 21 drehbar ist. Das Rohrstück 21
ist am vorderen Ende des Rohrkörpers 1 des Katheters fest
angebracht. Das andere Ende des Schildes 20 weist eine
senkrecht zur Katheterachse verlaufende Abbiegung 25 auf,
welche sich in einen Spalt 26 erstreckt. Der Spalt 26 befindet sich zwischen den beiden Teilen 19, 23 der Spitze 3
des Spreizkopfes 2 und wird von dem vorderen Ende des Spanndrahtes 7 durchsetzt. Der eine Teil 23 der geteilten Spitze 3
ist von einer Gewindemutter gebildet und der andere Teil von
einem mit Innengewinde versehenen Zylinder 19. In diese
Teile ist das mit Außengewinde versehene vordere Ende des
Spanndrahtes 7 eingeschraubt, jedoch unter Belassung eines definierten Spaltes 26. Infolge der festen Anbringung des Schutzschildes 20 macht dieser die Drehbewegung der Spitze 3 des

- 14 -

Spreizkopfes 2 sowie des Endstückes 4 nicht mit, so
daß über die Breite des Spaltes 26 genügend Spiel vorhanden ist, um dem Verschleiß zu begegnen.

Die Wirkungsweise der erfindungsgemäßen Vorrichtung ist
wie folgt:

Durch Anziehen des Spanndrahtes 7 aus seiner in Figur 6
dargestellten Ruhelage verkürzt sich der Abstand der Spitze
3 zum Endstück 4 mit der Folge einer Auswölbung der Schälmesser 6 in die in Figur 5 dargestellte Position unter
gleichzeitiger Auswölbung des bandförmigen Schutzschildes 20. Der Schutzschild 20 gewährleistet, daß an dem
durch ihn abgedeckten Bereich eine Gefäßwandung mit den
rotierenden Schälmessern 6 nicht in Berührung gelangen
kann, so daß einseitige Einschlüsse ohne die Gefahr einer
Beschädigung der Gefäßwandung beseitigt werden können.
Diese Ausgestaltung des Spreizkopfes findet beispielsweise
Anwendung bei dem vergleichsweise hohen Anteil einseitiger
Gefäßwandverkalkungen bei Patienten mit Durchblutungsstörungen der unteren Extremitäten.

Um bei Stenosen, die den ganzen Querschnitt eines Gefäßes
ausfüllen, die Schälmesser 6 des Spreizkopfes 2 zum Einsatz bringen zu können, wird durch die fräserartigen Rillen 22
auf der Umfläche des Teils 23 der Spitze 3 zunächst eine mittige Öffnung geschaffen, durch die der Spreizkopf 2 dann in
die Arbeitsposition der Schälmesser 6 gebracht werden kann.

0117519

- 15 -

Bei der Ausführung nach Figur 7 ist auf dem Rohrkörper 1 in seinem Bereich vor den Schälmessern 6 ein Schlauchstück 30 aus elastischem Material angebracht. In dem Überdeckungsbereich des Schlauchstückes 30 befindet sich eine Austrittsöffnung 31, die mit einem Kanal 32 in Verbindung steht, der sich in Längsrichtung des Rohrkörpers 1 durch dessen Wandung erstreckt. Bei Anschluß einer Druckmittelquelle, beispielsweise Luft oder Flüssigkeit, wird das Schlauchstück 30 aus seiner in Figur 9 gezeigten, am Rohrkörper 1 anliegenden Position ballonartig aufgewölbt, wie in Figur 7 dargestellt ist, und legt sich damit dichtend an die Innenwandung der umgebenden Vene, Arterie usw. an.

Bei der Ausführung nach Figur 8 ist die Spitze 3 geteilt ausgebildet. Die beiden Teile sind mit 33 und 34 bezeichnet. Auf dem Spanndraht 7 ist in seinem Bereich zwischen den beiden Teilen 33, 34 der Spitze 3 ein Schlauchstück 35 aus elastischem Material angebracht. Im Überdeckungsbereich des Schlauchstückes 35 ist die Austrittsöffnung 36 vorhanden, welche mit dem Kanal 37 in Verbindung steht, der sich in Längsrichtung des Spanndrahtes 7 erstreckt. Bei Beaufschlagung mit Druckmittel wölbt sich das Schlauchstück 35 ballonartig nach außen, wie aus Figur 8 ersichtlich ist, und legt sich an die Innenwandung einer nicht weiter dargestellten Vene, Arterie usw. dichtend an.

Im Rahmen der Erfindung ist es auch möglich, die Ausführungen nach Figur 7 und 8 miteinander zu kombinieren, so daß sowohl am vorderen als auch am hinteren Ende des Spreizkopfes sich je

- 16 -

ein Schlauchstück 30, 35 befindet mit der Möglichkeit einer
abschnittsweisen Abdichtung der Ausräumstelle. Nach Abschalten der Druckmittelbeaufschlagung nehmen die Schlauchstücke
30, 35 infolge der Elastizität ihres Materials die aus
Figur 9 ersichtliche Lage ein, so daß der Katheter um das
gewünschte Maß vor- oder zurückbewegt werden kann.

Der obere Teil 33 der Spitze kann als Fräskopf ausgebildet
sein, so daß dieser bei Einschlüssen, die einen wesentlichen
Teil des Gefäßquerschnittes einnehmen, einen Durchgang für
das Eingreifen der Schälmesser 6 schafft und daraufhin die Einschlüsse beseitigt werden können.

Durch Anziehen des Spanndrahtes 7 in seine aus den Figuren 7
und 8 ersichtliche Lage verkürzt sich der Abstand der Spitze 3
bzw. des hinteren Teils 4 der Spitze 3 zum Ende des Rohrstückes
1 mit der Folge einer Auswölbung der Schälmesser 6 in die gezeigte Position.

PATENTANSPRÜCHE :

1. Katheter zum Ausräumen von Einschlüssen in Venen, Arterien, Luft- und Speiseröhren sowie anderen Hohlorganen
des Körpers, welches einen biegsamen Rohrkörper aufweist,
dadurch gekennzeichnet, daß sich am vorderen Ende des Rohrkörpers (1) ein Spreizkopf (2) befindet, welcher ein oder
mehrere Schälmesser (6) aus hochelastischem Material aufweist, welches bzw. welche mittels eines in dem Rohrkörper (1)
verlaufenden Spanndrahtes (7) od.dgl. auswölbbar ist bzw. sind
und am hinteren Ende (10) des Rohrkörpers (1) ein Griff (5)
vorhanden ist.

2. Katheter nach Anspruch 1, dadurch gekennzeichnet, daß der
Spanndraht (7) od.dgl. sich von der Spitze (3) des Spreizkopfes (2) bis zum Griff (5) erstreckt.

3. Katheter nach Anspruch 1 oder 2, dadurch gekennzeichnet,
daß das bzw. die Schälmesser (6) sich mit ihren Enden einerseits an der Spitze (3) und andererseits an einem Endstück (4)
des Spreizkopfes (2) abstützen.

4. Katheter nach Anspruch 1 oder einem der vorhergehenden,
dadurch gekennzeichnet, daß im Rohrkörper (1) ein schraubenlinienförmig gewickeltes Band (8) vorgesehen ist, welches sich von der Spitze (3) des Spreizkopfes (2) bis zum
Griff (5) erstreckt.

5. Katheter nach Anspruch 1 oder einem der vorhergehenden, dadurch gekennzeichnet, daß das schraubenlinienförmige Band (8) den Spanndraht (7) od.dgl. umgibt.

6. Katheter nach Anspruch 1 oder einem der vorhergehenden, dadurch gekennzeichnet, daß die vorderen Enden des Spanndrahtes (7) od.dgl. und des schraubenlinienförmigen Bandes (8) mit der Spitze (3) des Spreizkopfes (2) drehfest und deren hintere Enden mit dem Griff (5) drehbar verbunden sind.

7. Katheter nach Anspruch 1 oder einem der vorhergehenden, dadurch gekennzeichnet, daß sich im Griff (5) ein Elektromotor befindet.

8. Katheter nach Anspruch 1 oder einem der vorhergehenden, dadurch gekennzeichnet, daß das hintere Ende (10) des Rohrkörpers (1) in ein mit Außengewinde versehenes Rohrstück (11) einmündet, welches von einem Rohrstutzen (12) übergriffen ist, welcher mit einer Gewindemuffe (13) versehen ist und über ein Zwischenstück (18) an den Griff (5) angeschlossen ist.

9. Katheter nach Anspruch 1 oder einem der vorhergehenden, dadurch gekennzeichnet, daß das Zwischenstück (18) in seiner Wandung eine oder mehrere Durchtrittsöffnungen (14) aufweist.

10. Katheter nach Anspruch 1 oder einem der vorhergehenden, dadurch gekennzeichnet, daß das Zwischenstück (18) mit einem Anschlußstutzen (17) ausgerüstet ist.

11. Katheter nach Anspruch 1 oder einem der vorhergehenden, dadurch gekennzeichnet, daß der Spreizkopf (2) mit einem etwa bandförmigen Schutzschild (20) aus hochelastischem Material ausgerüstet ist, welcher sich über einen Teil des Umfanges des Spreizkopfes (2) erstreckt.

12. Katheter nach Anspruch 1 oder einem der vorhergehenden, dadurch gekennzeichnet, daß das eine Ende des Schutzschildes (20) fest mit einem Rohrstück (21) verbunden ist, welches das Endstück (4) des Spreizkopfes (2) übergreift und an dem vorderen Ende des Rohrkörpers (1) des Katheters befestigt ist.

13. Katheter nach Anspruch 1 oder einem der vorhergehenden, dadurch gekennzeichnet, daß das andere Ende des Schildes (20) eine Abbiegung (25) senkrecht zur Katheterachse aufweist, welche sich in einen Spalt (26) zwischen den Teilen (19,23) der geteilt ausgebildeten Spitze (3) des Spreizkopfes (2) erstreckt und von dem Spanndraht (7) durchsetzt ist.

14. Katheter nach Anspruch 1 oder einem der vorhergehenden, dadurch gekennzeichnet, daß der eine Teil (23) der Spitze (3) von einer Gewindemutter gebildet ist und der andere Teil (19) von einem mit Innengewinde versehenen Zylinder (19).

15. Katheter zum Ausräumen von Einschlüssen in Venen, Arterien, Luft- und Speiseröhren sowie anderen Hohlorganen des Körpers, welches einen biegsamen Rohrkörper aufweist, an dessen vorderem Ende sich ein Spreizkörper befindet, welcher ein oder mehrere Schälmesser aus hochelastischem Material aufweist, welches bzw. welche mittels eines in dem Rohrkörper verlaufenden Spanndrahtes od.dgl. auswölbbar ist bzw. sind und am hinteren Ende des Rohrkörpers ein Griff vorhanden ist, wobei der
Spanndraht od.dgl. sich von der Spitze des Spreizkopfes
bis zum Griff erstreckt und das bzw. die Schälmesser
sich mit ihren Enden einerseits an der Spitze und andererseits an einem Endstück des Spreizkopfes abstützen,
dadurch gekennzeichnet, daß die Spitze (3) des Spreizkopfes (2) auf ihrer Umfläche mit fräserartigen Rillen
(22) od.dgl. versehen ist.

16. Katheter nach Anspruch 15, dadurch gekennzeichnet,
daß die fräserartigen Rillen schräg zur Katheterachse verlaufen.

17. Katheter nach Anspruch 1 oder einem der vorhergehenden,
dadurch gekennzeichnet, daß auf dem Rohrkörper (1) in seinem Bereich vor den Schälmessern (6) und/oder auf dem
Spanndraht (7) in seinem Bereich zwischen den beiden Teilen (33, 34) der geteilt ausgebildeten Spitze (3) des
Spreizkopfes (2) ein Schlauchstück (30, 35) aus elastischem Material angebracht ist bzw. sind, in dessen bzw.
deren Überdeckungsbereiche eine oder mehrere Austrittsöffnungen (31,36) von Kanälen (32,37) münden, die die
Wandung des Rohrkörpers (1) und/oder des Spanndrahtes (7)
in deren Längsrichtung durchsetzen.

18. Katheter nach Anspruch 1 oder einem der vorhergehenden,
dadurch gekennzeichnet, daß die Kanäle (32,37) mittels Druckmittel beaufschlagbar sind.

Fig.1

Fig.2

Fig.3

Fig.4

Fig. 6

Fig. 5

-4/4-

0117519

Fig. 9

Fig. 7

Fig. 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | GB-A-1 169 419  (EDWARDS LABORATORIES) <br> * Seite 1, Zeile 78 - Seite 2, Zeile 24; Figuren * | 1-3,11 ,12 | A 61 B  17/22 <br> A 61 B  17/32 |
| Y | | 4-10, 14-18 | |
| | --- | | |
| Y | US-A-3 320 957  (E. SOKOLIK) <br><br> * Spalte 2, Zeile 47 - Spalte 4, Zeile 16; Figuren * | 4-10, 14,17, 18 | |
| | --- | | |
| Y | US-A-3 937 222  (A. BANKO) <br> * Figuren 1-5A,10A * | 15,16 | |
| A | | 11,12 | RECHERCHIERTE SACHGEBIETE (Int. Cl. 3) |
| | --- | | A 61 B |
| A | US-A-2 730 101  (R.D. HOFFMAN) <br> * Figuren * | 1-3,8 | A 61 F |
| | --- | | |
| A | US-A-2 556 783  (F.J. WALLACE) <br> * Spalte 3, Zeilen 35-50; Figuren * | 1-3,8 | |
| | ---           -/- | | |

Der vorliegende Recherchenbericht wurde für alle Patentansprüche erstellt.

| Recherchenort <br> DEN HAAG | Abschlußdatum der Recherche <br> 29-05-1984 | Prüfer <br> WOLF C.H.S. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-4 273 128 (B.G. LARY)<br><br>* Spalte 3, Zeilen 6-39; Spalte 4, Zeilen 65-69; Figuren 1,2,10 *<br><br>--- | 1,3,17 ,18 | |
| A | DE-A-2 933 266 (D. HASSE)<br><br>* Figuren 3,5,8,11; Seite 12, Zeilen 27-31 *<br><br>--- | 1,2,11 ,12,15 -18 | |
| A | US-A-4 030 503 (W.T. CLARK III)<br>* Insgesamt *<br><br>--- | 1,4 | |
| A | DE-B-1 069 823 (A.J. METZ)<br><br>* Insgesamt *<br><br>----- | 1,17, 18 | |
| | | | **RECHERCHIERTE SACHGEBIETE (Int. Cl. 3)** |

Der vorliegende Recherchenbericht wurde für alle Patentansprüche erstellt.

| Recherchenort<br>DEN HAAG | Abschlußdatum der Recherche<br>29-05-1984 | Prüfer<br>WOLF C.H.S. |
|---|---|---|